# EUROPEAN PATENT SPECIFICATION

(11) **EP 2 915 504 B1**
(45) Date of publication and mention of the grant of the patent: **18.09.2019**
(21) Application number: 15275053.5
(22) Date of filing: 26.02.2015
(51) Int. Cl.: A61F 2/06, A61F 2/07, A61B 17/11

(54) **PROSTHESIS HAVING SHAPE MEMORY EFFECT FOR TREATING VASCULAR TRAUMA**
PROTHESE MIT FORMGEDÄCHTNISEIGENSCHAFT ZUR BEHANDLUNG EINES VASKULÄREN TRAUMAS
PROTHÈSE PRÉSENTANT UN EFFET DE MÉMOIRE DE FORME POUR LE TRAITEMENT DE TRAUMAS VASCULAIRES

(30) Priority: 03.03.2014 US 201461947242 P
(43) Date of publication of application: 09.09.2015
(73) Proprietor: Cook Medical Technologies LLC, Bloomington, IN 47404 (US)
(72) Inventor: Eaton, Elizabeth A., Bloomington, IN 47401 (US)
(74) Representative: Williams Powell

(56) References cited:
- US-A- 5 540 701
- US-A- 6 007 544
- US-A1- 2005 228 417
- US-A1- 2013 041 451

## Description

### BACKGROUND

### 1. Technical Field.

The present invention relates generally to medical prosthesis deployment systems for vascular repair. More particularly, the present disclosure relates to a prosthesis deployment system to repair a transected body vessel for gaining hemostasis during emergency medical procedures.

2. Background Information. Trauma physicians frequently encounter patients having traumatic injury to a body vessel, such as lacerated vessels or even transected vessels, resulting from gunshots, knife wounds, motor vehicle accidents, explosions, etc. Significant damage to a body vessel may expose a patient to deleterious conditions such as the loss of a limb, loss of function of a limb, increased risk of stroke, impairment of neurological functions, and compartment syndrome, among others. Particularly severe cases of vascular injury and blood loss may even result in death. In such severe situations, the immediate goal is to obtain hemostasis while maintaining perfusion of adequate blood flow to critical organs, such as the brain, liver, kidneys, and heart.

Examples of treatments that are commonly performed by trauma physicians to treat body vessel injuries include clamping the vessel with a hemostat, use of a balloon tamponade, ligation of the damaged vessel at or near the site of injury, or the insertion of one or more temporary shunts. However, conventional surgical repair is generally difficult with such actively bleeding, moribund patients. In many instances, there is simply not enough time to repair the body vessel adequately by re-approximating and suturing the body vessel. In many situations, the trauma physician will simply insert a temporary shunt (such as a Pruitt-Inahara Shunt) into the vessel. However, use of temporary shunts has been linked to the formation of clots. This may require returning the patient to the operating room for treatment and removal of the clots, often within about 36 to 48 hours of the original repair. Since shunts are generally placed as a temporary measure to restore blood flow and stop excessive blood loss, the shunt is typically removed when the patient has stabilized (generally a few days later) by a specialized vascular surgeon. After removal, the vascular surgeon will replace the shunt with a vascular graft, such as a fabric graft that is sewn into place. Ligation of the damaged blood vessel may result in muscle necrosis, loss of muscle function, or a potential limb loss or death.

Due to the nature of the body vessel injury that may be encountered, the use of shunts, repairing and/or ligating of a blood vessel often requires that such treatments be rapidly performed at great speed, and with a high degree of physician skill. Such treatments may occupy an undue amount of time and attention of the trauma physician at a time when other pressing issues regarding the patient's treatment require immediate attention. In addition, since the level of particularized skill required may exceed that possessed by the typical trauma physician, particularly traumatic episodes may require the skills of a physician specially trained to address the particular trauma, such as a vascular trauma, and to stabilize the patient in the best manner possible under the circumstances of the case.

Some open surgical techniques utilize sutures to affix damaged tissue portions surrounding fittings that have been deployed with the vessel, which requires the trauma physician to take time to tie the sutures properly. Although in modern medicine sutures can be tied in relatively rapid fashion, any step in a repair process that occupies physician time in an emergency situation is potentially problematic. In addition, the use of sutures to affix the vessel to the fitting compresses the tissue of the vessel against the fitting. Compression of tissue may increase the risk of necrosis of the portion of the vessel tissue on the side of the suture remote from the blood supply. When present, necrosis of this portion of the vessel tissue may result in the tissue separating at the point of the sutures. In this event, the connection between the vessel and the fitting may eventually become weakened and subject to failure. If the connection fails, the device may disengage from the vessel. Therefore, efforts continue to develop techniques that reduce the physician time required for such techniques, so that this time can be spent on other potentially life-saving measures.

US 6,007,544 discloses a catheter apparatus having a shape-memory alloy cuff and inflatable on-demand balloon for creating a bypass graft *in vivo.*

### SUMMARY OF THE PRESENT INVENTION

The present invention seeks to provide an improved prosthesis delivery system and an improved prosthesis.

The preferred embodiments described herein are able to provide a prosthesis deployment system for use in open surgical repair of an injured body vessel, such as an artery or a vein, (and in particular a transected vessel) during emergency surgery in a manner that is time-effective, that can address the trauma at hand to the extent possible, and that can utilize techniques that may be readily practiced by an trauma physician

According to an aspect of the present invention, there is provided a prosthesis delivery system as specified in claim 1.

According to another aspect of the present invention, there is provided a prosthesis as specified in claim 8.

A method of interconnecting a first vessel portion and a second vessel portion of a transected body vessel is disclosed. The method may include cooling a first end portion of a prosthesis. The first end portion may be radially movable between a compressed configuration and an expanded configuration. The first end portion may comprise a shape memory material having a transition temperature less than a body temperature such that the first end portion has an expanded configuration at a temperature above the transition temperature, and the first end portion has a compressed configuration at a temperature below the transition temperature. The cooling of the first end portion may be to a temperature below the transition temperature. The method may also include positioning the first end portion into the first vessel portion while the temperature is below the transition temperature.

### BRIEF DESCRIPTION OF THE DRAWINGS

Embodiments of the present invention are described below, by way of example only, with reference to the accompanying drawings, in which:
Fig. 1 is a side view of a prosthesis delivery system according to an embodiment of the present application;
Fig. 2 is a cross-sectional side view of the prosthesis delivery system of Fig. 1;
Fig. 3 is a cross-sectional side view of the prosthesis delivery system of Fig. 2 with cooling devices to cool the end portions;
Fig. 4 is a perspective view of a damaged portion of a body vessel;
Fig. 5 is a perspective view of the body vessel of Fig. 4 with the damaged portion transected and openings formed in healthy portions of the vessel that are adjacent to the transected portion;
Fig. 6 is a cross-sectional side view of the prosthesis delivery system of Fig. 2 showing sheaths removed from the prosthesis and the end portions of the prosthesis inserted into vessel portions of a patient while in a compressed configuration;
Fig. 7 is a cross-sectional side of the prosthesis delivery system of Fig. 6 with the end portions of the prosthesis in an expanded configuration;
Fig. 8 is a plot of fraction of martensite phase as a function of temperature for a shape memory alloy;
Fig. 9 is a perspective view of an example of a portion of a deployment system with a sheath on an end of a prosthesis according to an embodiment of the present application;
Fig. 10 is a longitudinal sectional view of the deployment system of Fig. 9; and
Fig. 11 is a transverse sectional view of the deployment system of Fig. 9.

### DESCRIPTION OF THE PREFERRED EMBODIMENTS

For the purposes of promoting an understanding of the teachings herein, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles illustrated therein being contemplated as would normally occur to one skilled in the art.

The prosthesis delivery systems described herein can be useful for open surgical repair of a body vessel, such as a blood vessel, during a medical procedure such as an emergency open surgical procedure. The prosthesis deployment systems can be particularly useful to deliver a prosthesis for repair of a lacerated artery or vein during emergency surgery, and particularly, to obtain hemostasis while maintaining blood perfusion, especially after transection of the body vessel.

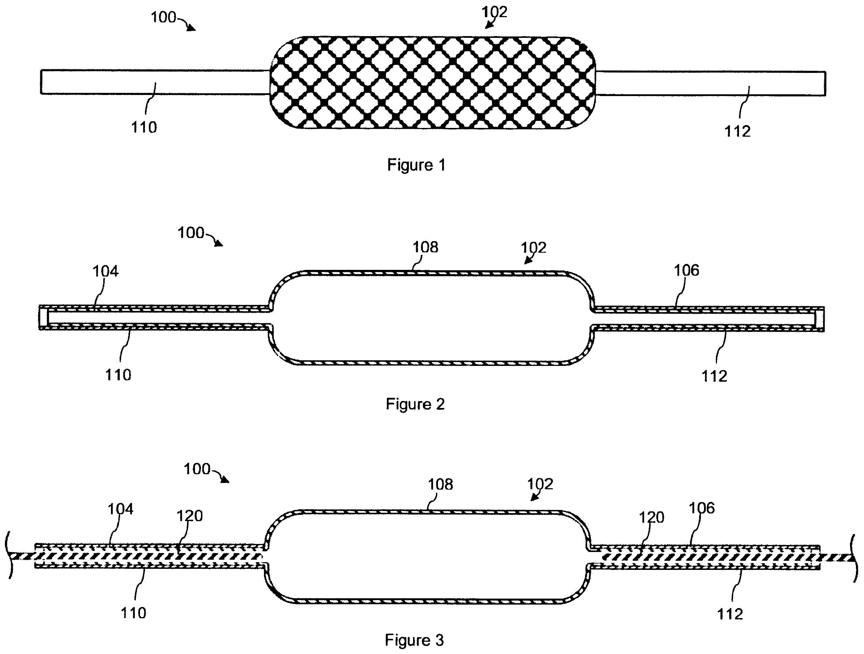

Fig. 1 is a side view and Fig. 2 is a cross-sectional side view that depict an example of a prosthesis delivery system 100 that includes a prosthesis 102. The prosthesis 102 may include a first end portion 104, a second end portion 106 and a central portion 108 between the first end portion 104 and the second end portion 106. The first end portion 104 may be radially movable between a compressed configuration and an expanded configuration. The second end portion 106 may also be radially movable between a compressed configuration and an expanded configuration. Figs. 1 and 2 depict the first end portion 104 and the second end portion 106 in a compressed configuration. The central portion 108 is depicted in an expanded configuration. The central portion 108 may be radially alterable between a compressed configuration and an expanded configuration.

The first end portion 104 may be maintained in a compressed configuration with a first sheath or sleeve 110. The first sheath 110 may prevent the first end portion 104 from radially expanding. Similarly, the second end portion 104 may be maintained in a compressed configuration with a second sheath or sleeve 112. The central portion 108 may not be in the compressed configuration. For example, a sheath may not maintain the central portion 108 in a compressed configuration, and the central portion 108 may be radially unrestricted. Thus, the central portion 108 may be configured to remain in an expanded configuration. As such, the system 100 may not include a sheath on the central portion 108.

The first end portion 104 may be or include a shape memory material having a transition temperature less than a body temperature such that the first end portion 104 has an expanded configuration at a temperature above the transition temperature, and the first end portion 104 has a compressed configuration at a temperature below the transition temperature. The second end portion 106 may also be or include a shape memory material having a transition temperature less than a body temperature such that the second end portion 106 has an expanded configuration at a temperature above the transition temperature, and the second end portion 106 has a compressed configuration at a temperature below the transition temperature.

Figs. 3-7 depict an example method of implanting the prosthesis 102 into a patient. Referring to Fig. 3, the delivery system 100 may include a cooling device 120 that can cool the first end portion 104 and/or the second end portion 106. If both the first end portion 104 and the second end portion 106 are cooled, the same cooling device 120 can be used or different cooling devices 120 may be used to cool each of the first end portion 104 and the second end portion 106. The cooling device 120 may cool the shape memory material to a temperature below the transition temperature of the shape memory material. By cooling the shape memory material to a temperature below the transition temperature, the first end portion 104 and/or the second end portion 106 may remain in the compressed configuration even without a radial restraint of the first sheath 110 and the second sheath 112, respectively. Thus, the first sheath 110 and/or the second sheath 112 may be removed after cooling the first end portion 104 and/or the second end portion 106, respectively.

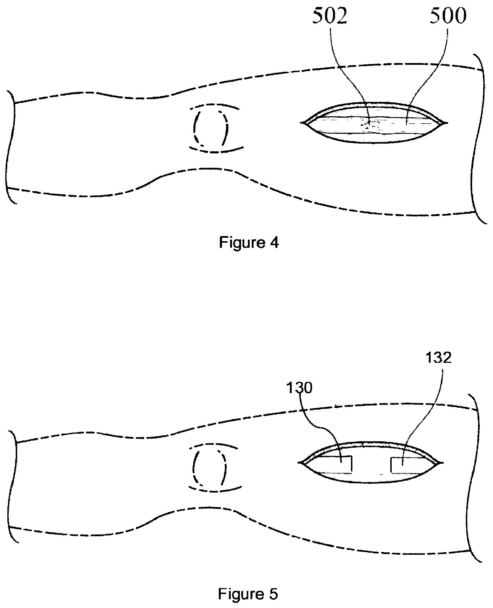

In Fig. 4, a body vessel 500, for example in the leg of a patient, has previously been subjected to a traumatic episode, which results in a portion 502 of body vessel 500 being torn away or otherwise severely damaged. Pre-surgery preparation has been applied to the leg and a trauma pathway may be formed therein in order to gain access to the body vessel and the damaged portion thereof. After clamping the body vessel 500 on both ends of the portion 502 to restrict blood flow temporarily, the blood vessel 500 can be cut or transected into two portions 130, 132 by the clinician, as shown in Fig. 5. The transection may be at the damaged portion 502 of the vessel 500 or as far away as necessary from the damaged portion to remove unhealthy portions of the body vessel or unrepairable portions of the body vessel. Sutures can be attached to the end openings of body vessel portions 130, 132 to keep them fixed in place and opened to facilitate insertion of the prosthesis. Forceps may also be used in a similar manner. The prosthesis 102 can be selected to have a radial expanded cross-section and a longitudinal length sufficient to bridge the body vessel portions 130, 132 and radially fit within the body vessel portions 130, 132.

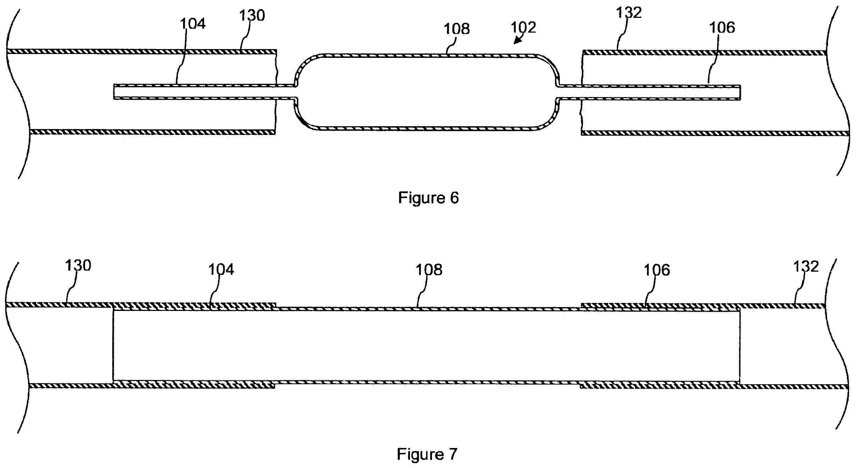

As depicted in Fig. 6, the prosthesis 102 can be positioned between the body vessel portions 130, 132 (target site) while the shape memory material has a temperature below the transition temperature. For example, the first end portion 104 may be positioned into a first vessel portion 130 while the temperature of the first end portion 104 is below the transition temperature. The second end portion 106 may be positioned into a second vessel portion 132 while the temperature of the second end portion 106 is below the transition temperature. After the prosthesis 102 has been positioned within the patient, the prosthesis 102 may warm up or increase in temperature as a result of heat from the patient transferring to the prosthesis 102. As depicted in Fig. 7, once the temperature of the first end portion 104 and/or the second end portion 106 raises to above the transition temperature of the shape memory material, the first end portion 104 and/or the second end portion 106 may radially expand and have the expanded configuration. Thus, the prosthesis can expand against the walls of the first vessel portion 130 and the second vessel portion 132 to provide a continuous lumen between the first vessel portion 130 and the second vessel portion 132.

The shape memory material can be one or more of various types such as alloys and polymers. One exemplary class of shape memory alloys is nickel-titanium (NiTi) alloys, also known as Nitinol. Nitinol is a metal alloy composition that includes nickel and titanium. However, Nitinol can also include various other alloying elements such as cobalt, chromium and/or erbium. For example, Nitinol can have a composition according to ASTM F2063. Upon cooling, nitinol transforms from having an austenite phase to having a martensite phase. The change from austenite to martensite is the reason that nitinol can have a shape-memory effect.

The shape-memory effect can be instilled into nitinol by deforming nitinol into a first shape while the crystal structure is austenite and cooling the nitinol to transform the austenite to martensite. The nitinol can be deformed into a second shape while the crystal structure is martensite. Upon reheating the nitinol to have the austenite crystal structure, the nitinol will change from the second shape to the first shape.

The shape-memory effect of the shape memory material can be one-way or two-way. For one-way memory effect, upon heating nitinol from martensite to austenite and the second shape changing to the first shape, the shape memory material will not change shape again upon further phase changes from additional heating or cooling. However, shape memory material can have two-way memory effect such that the shape memory material can go through a plurality of martensite to austenite to martensite phase changes with shape changes still occurring through each martensite to austenite and austenite to martensite phase change. Thus, the shape memory material with two way memory effect can have at least a first shape while martensite and a second shape while austenite and maintain the distinct shapes even after a plurality of phase change cycles between martensite and austenite. Thus, in contrast to one-way memory effect in which the shape memory alloy must, after being heated to the austenite phase, be mechanically deformed while in the martensite phase to re-impart a shape memory effect, two-way can maintain the shape memory effect, after being heated to the austenite phase, without having to be mechanically deform again. In other words, two-way effect exhibits shape change upon both cooling and heating while one-way effect only exhibits shape change upon heating.

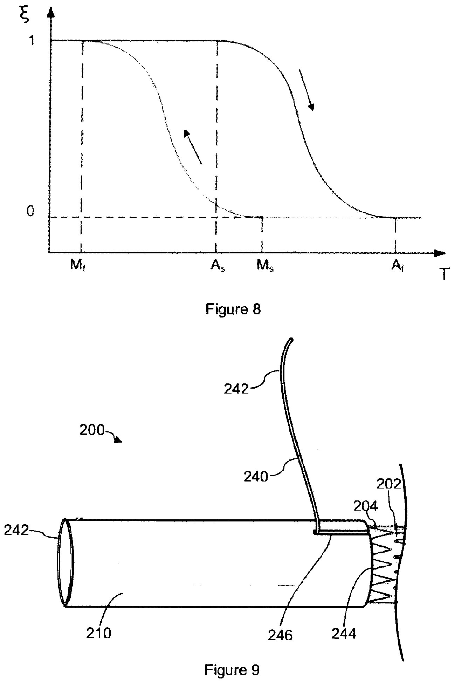

In addition, transition between martensite and austenite does not typically occur at a specific temperature but instead, over a temperature range. Fig. 8 is a plot of fraction of martensite as a function of temperature. Upon heating from martensite to austenite, austenite begins to form at temperature Aₛ and completes transformation to austenite at temperature A_{f}. Upon cooling from martensite to austenite, martensite beings to form at temperature Mₛ and completes transformation to martensite at temperature M_{f}. As shown in Fig. 8, the cooling and heating cycle shows thermal hysteresis as a result of mechanical energy lost in the process. Thus, temperature Aₛ is not necessarily equal to temperature M_{f}, and temperature A_{f} is not necessarily equal to temperature Mₛ. Thus, the transition temperature may actually be a temperature range. However, transition temperature as used herein refers to a temperature in which at least some change in shape due to the shape memory effect occurs upon heating from a first temperature to a second temperature. For example, complete phase change from martensite to austenite upon heating may not be necessarily for the end portions 104, 106 of the prosthesis 102 to change from the compressed configuration to the expanded configuration. Thus, the transition temperature can be a temperature from Aₛ to A_{f}. In one example, the transition temperature may be equal to A_{f} so that after the end portions 104, 106 of the prosthesis 102 warm to body temperature, the end portions 104, 106 are substantially or completely austenite. Therefore, in an expanded configuration, the end portions 104, 106 of the prosthesis 102 may be primarily, substantially or completely austenite. In a compressed configuration, the end portions 104, 106 of the prosthesis 102 may be primarily, substantially or completely martensite when cooled below the transition temperature. However, as described above, the end portions 104, 106 may be retained with a sheath 110, 112 in a collapsed configuration. Therefore, when end portions 104, 106 are retained, the end portions 104, 106 may be primarily, substantially or completely austenite.

Furthermore, the transition temperature can be selected by selecting a particular alloy composition. For example, the M_{f}, Aₛ, Mₛ and/or A_{f} can be selected by selecting a nitinol composition that is either nickel rich or titanium rich from stoichiometric NiTi. As described above, the transition temperature can be less than the body temperature. Thus, the transition temperature can be less than 37 °C. However, the transition temperature may be further away from the body temperature such as less than about 36 °C or less than about 30 °C. However, the closer the transition temperature is to body temperature the less the end portion may be cooled. Thus, the transition temperature may be greater than about 20 °C or greater than about 30 °C.

The shape memory alloy may be super cooled below the transition temperature to provide sufficient time between cooling of the shape memory alloy and implanting the prosthesis 102 so that the shape memory alloy does not warm to a temperature equal to or greater to the transition temperature prior to implantation. For example, the shape memory alloy may be cooled to a temperature of at least about 10 °C below the transition temperature or at least about 20 °C below the transition temperature. However, if cooled to too low of a temperature, the cooled prosthesis may cause damage to tissue in the patient. Thus, the shape memory alloy may be cooled to a temperature between about 10 °C and about 30 °C below the transition temperature.

When the shape memory alloy has two-way memory effect, the sheath may not be necessary. For example, at a temperature above the transition temperature, the first end portion 104 and/or the second end portion 106 may have the expanded configuration, and at a temperature below the transition temperature, the first end portion 104 and/or the second end portion 106 may have the compressed configuration. Therefore, upon cooling the first end portion 104 and/or the second end portion 106 below the transition temperature, the first end portion 104 and/or the second end portion 106 will adopt the compressed configuration without having to apply an inward radial force to the first end portion 104 and/or the second end portion 106. Thus, in order to have the first end portion 104 and/or the second end portion 106 in the compressed configuration, the first end portion 104 and/or the second end portion 106 may be cooled to below the transition temperature.

The central portion 108 may also be a shape memory material. However, the central portion 108 does not necessarily need to be configured to be able to have a compressed configuration. Therefore, the central portion 108 may not be or comprise a shape memory material, and instead, the central portion 108 may be or comprise a non-shape memory material. For example, the central portion 108 may be or comprise a different material than the first end portion 104 and/or the second end portion 106.

The cooling device 120 can include a probe or rod inserted during manufacture or configured to be inserted in the field into luminal space of the first end portion 104 and/or the second end portion 106 in a compressed configuration. The probe may be cooled prior to being inserted into the luminal space. For example, probe may be cooled with aerosel. In another example, the probe may be placed in a pouch along with a cold-pack. Alternatively, the probe itself may also provide the cooling such as the probe may include chemicals that can provide an endothermic reaction when mixed. The cooling device 120 may not be inserted into the luminal space, and instead, cooling can be applied to the exterior of the first end portion 104 and/or the second end portion 106. However, the sheath 110, 112 may decrease cooling rate, and therefore, cooling from within the luminal space may result in a faster cooling rate.

The sheath 110, 112 can be removed by sliding, cutting or peeling the sheath off of the end portion 104, 106 of the prosthesis 102. As described below, the end portion 104, 106 may include an anchoring member which may restrict the sheath 110, 112 from sliding off of the end portion 104, 106. Therefore, the sheath 110, 112 may be configured to split. In addition, after cooling of the end portions 104, 106 of the prosthesis 102, the prosthesis 102 is to be inserted into the vessel portions 130, 132 before the end portions 104, 106 have warmed to a temperature above the transition temperature. Therefore, the sheath 110, 112 can be configured to be removed quickly. In another example, the sheath 110, 112 may be slid toward a center region of the prosthesis 102. After the sheath 110, 112 has been slid toward the center region of the prosthesis 102, the anchoring members may be exposed, and the prosthesis 102 can be engaged with the vessel portion 130, 132. After the prosthesis 102 has been engaged with the vessel portion 130, 132, the sheath 110, 112 can be removed such as cutting or peeling away the sheath 110, 112 from the prosthesis 102.

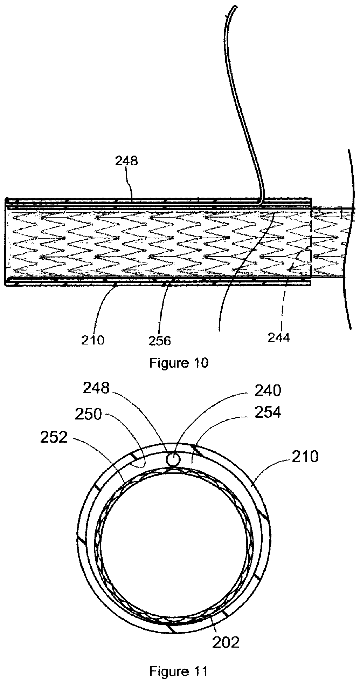

Figs. 9-11 depict one example of a sheath system 200 for removing a sheath 210 from the end portion 204. The sheath system 200 can include a sheath 210 and a splitting member 240. In some embodiments, the sheath system 200 is similar to the deployment systems described in United States Patent Publication No. 2013/0041451. As depicted in Figs. 9 and 10, the sheath system 200 includes a sheath 210 on the end portion 204 of the prosthesis 202 for controlling the expansion at the end portion 204. A sheath system 200 can be used to retain each of the end portions 204 of the prosthesis 202. The sheath 210 can include a tubular body extending between an outer end 242 and an inner end 244. The splitting member 240 may be coupled to the sheath 210. The splitting member 240 can have an outer end portion 246 extending from the inner end 244 of the sheath 210. The splitting member 240 can also extend beyond the outer end 242 of the sheath 210. An intermediate portion 248 can be disposed between the respective end portions of the splitting member 240.

The sheath 210 can be fitted over the end portion 204 of the prosthesis 202. The sheath 210 can be in a non-split configuration to retain the end portions 204 of the prosthesis 202 in a radially compressed configuration for cooling. A lumen can extend through the sheaths 210 and can be sized to receive the prosthesis 202 in the compressed configuration.

As described above, the splitting member 240 can have outer end 242 extending outwardly from the inner end 244 of the sheath 210. The outer end 242 of the splitting member 240 can be retracted toward outer end 242 of the prosthesis 202 from the inner end 244 and may be further retracted longitudinally away from the outer end of the prosthesis 202. When retracted, the splitting member 240 can split or cut through the wall of the sheath 210 in an inside-out direction. The sheath 210 can be in a split configuration to allow for expansion of the end portion 204 of the prosthesis 202 to a radially expanded configuration. The splitting member 240 can also be configured to split the prosthesis sheath 210 from the outer end 242 toward the inner end 244.

The axial length of the sheath 210 and the end portion 204 can be coextensive or different from each other. For example, sheath 210 may have an axial length so that the outer end 242 extends outwardly beyond the outer end of the prosthesis 202 by a distance, such as about 1 cm.

The sheath 210 may have at least one slit formed therein to allow the passage of the splitting member 240 and guide the splitting action with the splitting member 240. In Figs. 9 and 10, a slit 246, e.g., shaped as an axial slit, can be formed in the wall of the sheath 210 to extend from the inner end 244 of the sheath 210 inward by a length. The slit may terminate at a position between the inner end 244 and the outer end 242 of the sheath 210. For example, the slit 246 may terminate at a position between the outer end of the prosthesis 204 and the inner end 244 of the retainer sheath 210 to ensure that a ring of sheath material surrounds the prosthesis 202 for facilitating its compressed configuration.

In Fig. 11, a segment, such as an intermediate portion 248 of the splitting member 240, can be disposed within the lumen of the retainer sheath 210, sandwiched between the luminal wall 250 of the sheath 210 and the outer surface 252 of the prosthesis 202. The intermediate portion 248 of the splitting member 240 can extend axially within an annular space 254 between the sheath 210 and the prosthesis 202. The splitting member 240 can have a tensile strength sufficient to be pulled during the splitting action without breaking. The splitting member 240 may be formed generally as a flexible elongated member such as, e.g., a metal wire, plastic strip, a suture, or the like. In one example, the splitting member is a stainless steel, copper, or nitinol wire having a diameter of about 0.25 mm (0.01 inches). The outer end portion 242 of the splitting member 240 can be an enlarged end for improved grippability by the end user during retraction of the splitting member. For example, the outer end portion 242 may include a handle.

Fig. 10 depicts the system 200 provided with an inner barrier segment 256 disposed within the sheath 210 between the prosthesis 202 and the splitting member 240. The barrier segment 256 can prevent the prosthesis 202, such as the graft body or the support structure, from being compromised or otherwise damaged with the movement of the splitting member 240 during the cutting action. The length of the barrier segment 256 can be sufficient to extend at least partially between the outer and inner ends of each of the sheaths 210. Additional configurations of the barrier segment 170 are described in United States Patent Publication No. 2013/0041451.

The sheath and/or the barrier segment described herein can be constructed from one or more biocompatible polymeric layers. For example, the sheath and/or segment can be extruded from a biocompatible polymer material. In addition, the sheath and/or segment can be formed of at least one layer such as a polyether block amide, nylon, polyurethane, polytetrafluoroethylene (PTFE), FEP, or any combination thereof. The sheath and/or the barrier segment can be configured to be separated, preferably longitudinally, along a relatively predictable path. The material of the retainer sheath is configured to be split or cut into two or more portions by movement of the splitting member, thereby opening a fissure along the length that permits its removal from around the prosthesis situated therein. A predetermined split line may be formed in the sheath and/or the barrier segment through which the tear or split progresses due to properties of, and/or features incorporated into the material. When present, the predetermined split line can withstand being subjected to a curve to the degree required by the particular application without kinking or premature separation. In one example, the sheath can comprise a splittable polymer such as molecularly oriented, non-isotropic PTFE that is used to make the PEEL-AWAY® Introducer Sheath, which is commercially provided by Cook Medical Inc. (Bloomington, Ind.). Such sheath is described in, e.g., U.S. Patent 4,306,562 to Osborne and U.S. Patent 4,581,025 to Timmermans. In other examples, the sheath can include one or more pre-weakened features, such as a score line, perforations, or reduced wall thickness regions, extending longitudinally along the length of the sheath.

The prosthesis 102 can be any type of implant, stent, graft or conduit that is used for medical applications, and an exemplary prosthesis is shown in the Figures. The prosthesis can include a generally tubular graft portion and one or more stent structures that are attached to the graft. The prosthesis 102 can be expandable between the radially compressed, delivery configuration that is shown in Fig. 1, to the radially expanded, deployed configuration. The stent structure can be attached to an outer surface of the graft so that a lumen of the graft may provide a clear path for fluid flow, and/or attached to the inner surface of the graft. The prosthesis can be sized and shaped for suitable placement within a body vessel, such as an artery or vein, and particularly, for placement at the site of a vascular trauma such as a transected vessel. The stent structure can be any pattern of stent structures in the art that are designed primarily for vascular applications, and can be self-expanding.

An anchoring member can be disposed along any portion of the prosthesis for securely engaging the vessel wall in order to inhibit migration of the prosthesis after deployment or detachment of the vessel wall from the prosthesis. Preferably, the anchoring member is disposed along the two end portions of the prosthesis. The anchoring member can include barbs or various shaped member structures, including fibers, bristles, or outer protruding and penetrable media.

The graft can be a liner that extends partially or entirely along the luminal wall, abluminal wall, or both the luminal and abluminal walls of the stent structure. The graft can be made of material to inhibit fluid or blood located within the prosthesis lumen from passing through the graft. In other words, fluid flow is urged by the graft to enter into one end and exit out of the other end of the prosthesis. The graft can be formed from conventional materials well known in the medical arts. It is preferred that the graft covering have a porosity (e.g., about 10 to about 150 µm) for sufficient capillarization and be relatively thin as possible (e.g., about 0.127 to 0.254 mm (0.005 to 0.010 inch) and preferably about 0.0254 to about 0.0889 mm (about 0.001 to 0.0035 inch)). Examples of pore density and pore size for the graft covering, as well as other types of materials for a graft covering can be found in U.S. Patent 7,244,444 to Bates. A particularly preferred material is expanded polytetrafluoroethylene (ePTFE). Other materials that may be suitable in a particular case include, among others, polytetrafluoroethylene, silicone, polyurethane, polyamide (nylon), as well as other flexible biocompatible materials. Graft covering can also be formed from known fabric graft materials such as woven polyester (e.g. DACRON®), or from a bioremodelable material. One exemplary graft material is THORALON® from Thoratec Corporation (Pleasanton, Calif.), that can prevent leakage of fluid through the pores of the graft. THORALON® is a polyetherurethane urea blended with a siloxane containing surface modifying additive, and has been demonstrated to provide effective sealing of textile grafts. Another example is polyethylene, and in particular, an ultra-high molecular weight polyethylene (UHMwPE), commercially available as DYNEEMA®. The graft may also include a bioremodelable material that can provide an extracellular matrix that permits, and may even promote, cellular invasion and ingrowth into the material upon implantation. Non-limiting examples of suitable bioremodelable materials include reconstituted or naturally-derived collagenous materials. Suitable collagenous materials may include an extracellular matrix material (ECM) that possesses biotropic properties, such as submucosa, renal capsule membrane, dermal collagen, dura mater, pericardium, fascia lata, serosa, peritoneum or basement membrane layers. Suitable submucosa materials may include, for example, intestinal submucosa, including small intestinal submucosa (SIS), stomach submucosa, urinary bladder submucosa, and uterine submucosa. One non-limiting example of a suitable remodelable material is the SURGISIS® BIODESIGN™, which is commercially available from Cook Medical Inc. (Bloomington, Ind.). Another suitable remodelable material is the graft prosthesis material described in U.S. Patent No. 6,206,931 to Cook et al.,. The remodelable material can be ECM, SIS, remodelable or collagenous foam, foamed ECM, lyophilized SIS, vacuum pressed SIS, or the like.

The prosthesis can also include a coating of one or more therapeutic agents along a portion of the conduit body and/or the graft coverings. Therapeutic agents for use as biocompatible coatings are well known in the art. Non-limiting examples of suitable bio-active agents that may be applied to the vascular conduit include thrombo-resistant agents, antibiotic agents, anti-tumor agents, antiviral agents, anti-angiogenic agents, angiogenic agents, anti-mitotic agents, anti-inflammatory agents, angiostatin agents, endostatin agents, cell cycle regulating agents, genetic agents, including hormones such as estrogen, their homologs, derivatives, fragments, pharmaceutical salts and combinations thereof. Those skilled in the art will appreciate that other bioactive agents may be applied for a particular use. The bioactive agent can be incorporated into, or otherwise applied to, portions of the vascular conduit by any suitable method that permits adequate retention of the agent material and the effectiveness thereof for its intended purpose.

An exemplary method of interconnecting a first vessel portion and a second vessel portion of a transected body vessel, includes the following steps: cooling a first end portion of a prosthesis, the first end portion being radially movable between a compressed configuration and an expanded configuration, the first end portion comprising a shape memory material having a shape memory material having a transition temperature less than a body temperature such that the first end portion has an expanded configuration at a temperature above the transition temperature, and the first end portion has a compressed configuration at a temperature below the transition temperature, wherein the cooling the first end portion is to a temperature below the transition temperature; and positioning the first end portion into the first vessel portion while the temperature is below the transition temperature.

The method may comprising warming the first end portion to a temperature above the transition temperature after positioning the first end portion into the first vessel portion.

Advantageously, a central portion of the prosthesis that is adjacent to the first end portion that remains in an expanded configuration during the positioning of the first end portion into the first vessel portion.

The method may also comprise: cooling the second end portion of a prosthesis, the second end portion being radially movable between a compressed configuration and an expanded configuration, the second end portion comprising a shape memory material having a shape memory material having a transition temperature less than a body temperature such that the second end portion has an expanded configuration at a temperature above the transition temperature, and the second end portion has a compressed configuration at a temperature below the transition temperature, wherein the cooling the second end portion is to a temperature below the transition temperature; and positioning the second end portion into the second vessel portion while the temperature is below the transition temperature.

The method may also provide for a central portion between the first end portion and the second end portion that remains in an expanded configuration during the positioning of the first end portion into the first vessel portion and the second end portion into the second vessel portion.

During the position of the first end portion into the first vessel portion, the first end portion may not be actively cooled.

The method may also comprise removing a sheath that retains the first end portion in the compressed configuration after cooling the first end portion and prior to positioning the first end portion into the first vessel portion.

Removing the sheath may comprise retracting a portion of a splitting member associated with the sheath to split a wall of the sheath.

Although the prosthesis and the deployment system has been described in connection with its primary intended use for repair of vascular trauma, those skilled in the art will appreciate that the device may also be used to repair other traumatic conditions. Non-limiting examples of such conditions include aneurysms, such as abdominal aorta aneurysms, and surgery for tumor removal. In another matter of terminology there are many types of body canals, blood vessels, ducts, tubes, and other body passages, and the term "body vessel" is meant to include all such passages. Other vascular applications include coronary arteries, carotid arteries, vascular aneurysms, and peripheral arteries and veins (e.g., renal, iliac, femoral, popliteal, subclavian, aorta, intracranial, etc.). Other nonvascular applications include gastrointestinal, duodenum, biliary ducts, esophagus, urethra, reproductive tracts, trachea, and respiratory (e.g., bronchial) ducts. To this end, the deployment systems and methods described herein can be used to deliver a prosthesis to any of these vessels, ducts, canals, tubes or body passageways.

While preferred embodiments of the invention have been described, it should be understood that the invention is not so limited, and modifications may be made without departing from the invention. The scope of the invention is defined by the appended claims, and all devices that come within the meaning of the claims, either literally or by equivalence, are intended to be embraced therein. Furthermore, the advantages described above are not necessarily the only advantages of the invention, and it is not necessarily expected that all of the described advantages will be achieved with every embodiment of the invention.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A prosthesis delivery system (100) for repair of a body vessel including:
a tubular prosthesis (102; 202) including:
a first end portion (104) being radially movable between a compressed configuration and an expanded configuration, the first end portion comprising a shape memory material having a transition temperature less than a body temperature such that the first end portion has an expanded configuration at a temperature above the transition temperature and a compressed configuration at a temperature below the transition temperature;
a second end portion (106) being radially movable between a compressed configuration and an expanded configuration;
a central portion (108) between the first end portion and the second end portion; and
a first sheath (110; 210) that retains the first end portion in a compressed configuration, **characterized in that** the central portion is radially unrestricted by the sheath so as not to be in a compressed configuration.

2. A delivery system (100) according to claim 1, wherein the second end portion (106) comprises a shape memory material having a transition temperature less than a body temperature such that the second end portion has an expanded configuration at a temperature above the transition temperature, and the second end portion has a compressed configuration at a temperature below the transition temperature.

3. A delivery system (100) according to claim 2, including a second sheath (112) that retains the second end portion (106) in a compressed configuration.

4. A delivery system (100) according to any preceding claim, wherein a splitting member (240) is associated with the first sheath (110; 210) and is operable to split a wall of the sheath.

5. A delivery system (100) according to any preceding claim, including a cooling device (120) to cool the first end portion (104) to a temperature below the transition temperature of the first end portion.

6. A delivery system (100) according to claim 5, wherein the cooling device (120) is configured to not cool the central portion (108) of the prosthesis.

7. A delivery system according (100) to any preceding claim, wherein the central portion (108) is formed from a non-shape memory material.

8. A tubular prosthesis (102; 202) for repair of a body vessel, the tubular prosthesis is having an expanded configuration having a radially expanded cross-section, including:
a first end portion (104) being radially movable between a compressed configuration and the expanded configuration, the first end portion comprising a shape memory material having a transition temperature less than a body temperature such that the first end portion has the expanded configuration at a temperature above the transition temperature and a compressed configuration at a temperature below the transition temperature;
a second end portion (106) being radially adjustable between a compressed configuration and the expanded configuration; and
a central portion (108) between the first end portion and the second end portion, **characterized in** the central portion being configured to remain in the expanded configuration whilst the first end portion is in its compressed configuration.

9. A prosthesis (102; 202) according to claim 8, wherein the central portion (108) is a non-shape memory material.

10. A prosthesis (102; 202) according to claim 8 or 9, wherein the first end portion (104) includes at least one anchoring member.

11. A prosthesis (102; 202) according to claim 8, 9 or 10, wherein the first end portion (104) is or comprises Nitinol.

## Patentansprüche

1. Prothesenzuführsystem (100) zur Reparatur eines Körpergefäßes, umfassend:
eine röhrenförmige Prothese (102; 202), die umfasst:
einen ersten Endabschnitt (104), der zwischen einer komprimierten Auslegung und einer aufgeweiteten Auslegung radial beweglich ist, wobei der erste Endabschnitt ein Formgedächtnismaterial umfasst, das eine Übergangstemperatur aufweist, die geringer ist als eine Körpertemperatur, sodass der erste Endabschnitt eine aufgeweitete Auslegung mit einer Temperatur über der Übergangstemperatur und eine komprimierte Auslegung mit einer Temperatur unter der Übergangstemperatur aufweist;
einen zweiten Endabschnitt (106), der zwischen einer komprimierten Auslegung und einer aufgeweiteten Auslegung radial beweglich ist;
einen zentralen Abschnitt (108) zwischen dem ersten Endabschnitt und dem zweiten Endabschnitt; und
eine erste Hülle (110; 210), die den ersten Endabschnitt in einer komprimierten Auslegung hält, **dadurch gekennzeichnet, dass** der zentrale Abschnitt durch die Hülle radial uneingeschränkt ist, um nicht in einer komprimierten Auslegung zu sein.

2. Zuführsystem (100) nach Anspruch 1, wobei der zweite Endabschnitt (106) ein Formgedächtnismaterial umfasst, das eine Übergangstemperatur aufweist, die geringer ist als eine Körpertemperatur, sodass der zweite Endabschnitt eine aufgeweitete Auslegung mit einer Temperatur über der Übergangstemperatur aufweist und der zweite Endabschnitt eine komprimierte Auslegung mit einer Temperatur unter der Übergangstemperatur aufweist.

3. Zuführsystem (100) nach Anspruch 2, umfassend eine zweite Hülle (112), die den zweiten Endabschnitt (106) in einer komprimierten Auslegung hält.

4. Zuführsystem (100) nach einem vorhergehenden Anspruch, wobei ein Spaltelement (240) mit der ersten Hülle (110; 210) verknüpft ist und betrieben wird, um eine Wand der Hülle aufzuspalten.

5. Zuführsystem (100) nach einem vorhergehenden Anspruch, umfassend eine Kühleinrichtung (120), um den ersten Endabschnitt (104) auf eine Temperatur unter der Übergangstemperatur des ersten Endabschnitts zu kühlen.

6. Zuführsystem (100) nach Anspruch 5, wobei die Kühleinrichtung (120) dazu ausgelegt ist, den zentralen Abschnitt (108) der Prothese nicht zu kühlen.

7. Zuführsystem (100) nach einem vorhergehenden Anspruch, wobei der zentrale Abschnitt (108) nicht aus Formgedächtnismaterial gebildet ist.

8. Röhrenförmige Prothese (102; 202) zur Reparatur eines Körpergefäßes, wobei die röhrenförmige Prothese eine aufgeweitete Auslegung mit einem radial aufgeweiteten Querschnitt aufweist, umfassend:
einen ersten Endabschnitt (104), der zwischen einer komprimierten Auslegung und der aufgeweiteten Auslegung radial beweglich ist, wobei der erste Endabschnitt ein Formgedächtnismaterial umfasst, das eine Übergangstemperatur aufweist, die geringer ist als eine Körpertemperatur, sodass der erste Endabschnitt die aufgeweitete Auslegung mit einer Temperatur über der Übergangstemperatur und eine komprimierte Auslegung mit einer Temperatur unter der Übergangstemperatur aufweist;
einen zweiten Endabschnitt (106), der zwischen einer komprimierten Auslegung und der aufgeweiteten Auslegung radial einstellbar ist; und
einen zentralen Abschnitt (108) zwischen dem ersten Endabschnitt und dem zweiten Endabschnitt, **gekennzeichnet durch** den zentralen Abschnitt, der dazu ausgelegt ist, in der aufgeweiteten Auslegung zu bleiben, während der erste Endabschnitt in seiner komprimierten Auslegung ist.

9. Prothese (102; 202) nach Anspruch 8, wobei der zentrale Abschnitt (108) kein Formgedächtnismaterial ist.

10. Prothese (102; 202) nach Anspruch 8 oder 9, wobei der erste Endabschnitt (104) mindestens ein Verankerungselement umfasst.

11. Prothese (102; 202) nach Anspruch 8, 9 oder 10, wobei der erste Endabschnitt (104) Nitinol ist oder umfasst.

## Revendications

1. Système de distribution de prothèse (100) pour la réparation d'un vaisseau corporel comprenant :
une prothèse tubulaire (102 ; 202) comprenant :
une première partie d'extrémité (104) mobile radialement entre une configuration comprimée et une configuration expansée, la première partie d'extrémité comprenant un matériau à mémoire de forme ayant une température de transition inférieure à une température du corps de telle sorte que la première partie d'extrémité a une configuration expansée à une température supérieure à la température de transition et une configuration comprimée à une température inférieure à la température de transition ;
une seconde partie d'extrémité (106) mobile radialement entre une configuration comprimée et une configuration expansée ;
une partie centrale (108) entre la première partie d'extrémité et la seconde partie d'extrémité ; et
une première gaine (110 ; 210) qui retient la première partie d'extrémité dans une configuration comprimée, **caractérisé en ce que** la partie centrale est radialement non limitée par la gaine de manière à ne pas être dans une configuration comprimée.

2. Système de distribution (100) selon la revendication 1, la seconde partie d'extrémité (106) comprenant un matériau à mémoire de forme ayant une température de transition inférieure à une température du corps de telle sorte que la seconde partie d'extrémité a une configuration expansée à une température supérieure à la température de transition, et la seconde partie d'extrémité a une configuration comprimée à une température inférieure à la température de transition.

3. Système de distribution (100) selon la revendication 2, comprenant une deuxième gaine (112) qui retient la seconde partie d'extrémité (106) dans une configuration comprimée.

4. Système de distribution (100) selon n'importe quelle revendication précédente, un élément de fendage (240) étant associé à la première gaine (110 ; 210) et pouvant être actionné pour fendre une paroi de la gaine.

5. Système de distribution (100) selon n'importe quelle revendication précédente, comprenant un dispositif de refroidissement (120) pour refroidir la première partie d'extrémité (104) à une température inférieure à la température de transition de la première partie d'extrémité.

6. Système de distribution (100) selon la revendication 5, le dispositif de refroidissement (120) étant configuré pour ne pas refroidir la partie centrale (108) de la prothèse.

7. Système de distribution (100) selon n'importe quelle revendication précédente, la partie centrale (108) étant formée d'un matériau sans mémoire de forme.

8. Prothèse tubulaire (102 ; 202) pour la réparation d'un vaisseau corporel, la prothèse tubulaire ayant une configuration expansée ayant une section transversale expansée radialement, comprenant :
une première partie d'extrémité (104) mobile radialement entre une configuration comprimée et la configuration expansée, la première partie d'extrémité comprenant un matériau à mémoire de forme ayant une température de transition inférieure à une température du corps de telle sorte que la première partie d'extrémité ait la configuration expansée à une température supérieure à la température de transition et une configuration comprimée à une température inférieure à la température de transition ;
une deuxième partie d'extrémité (106) radialement ajustable entre une configuration comprimée et la configuration expansée ; et
une partie centrale (108) entre la première partie d'extrémité et la deuxième partie d'extrémité, **caractérisée en ce que** la partie centrale est configurée pour rester dans la configuration expansée tandis que la première partie d'extrémité est dans sa configuration comprimée.

9. Prothèse (102 ; 202) selon la revendication 8, la partie centrale (108) étant un matériau sans mémoire de forme.

10. Prothèse (102 ; 202) selon la revendication 8 ou 9, la première partie d'extrémité (104) comprenant au moins un élément d'ancrage.

11. Prothèse (102 ; 202) selon la revendication 8, 9 ou 10, la première partie d'extrémité (104) étant ou comprenant du Nitinol.
